# EUROPEAN PATENT SPECIFICATION

(11) **EP 1 904 217 B1**
(45) Date of publication and mention of the grant of the patent: **27.03.2013**
(21) Application number: 05773059.0
(22) Date of filing: 19.07.2005
(51) Int. Cl.: A61F 2/01

(54) **EMBOLIC FILTERING METHOD AND APPARATUS**
EMBOLIEFILTERVERFAHREN UND VORRICHTUNG
PROCEDE ET APPAREIL DE FILTRAGE EMBOLIQUE

(43) Date of publication of application: 02.04.2008
(73) Proprietor: SeptRx, Inc., Fremont, CA 94539 (US)
(72) Inventor: KLESHINSKI, Stephen, J., Scituate, MA 02066 (US); RUSSELL, Scott, M., San Jose, CA 95125 (US)
(74) Representative: Price, Nigel John King
(86) International application number: PCT/US2005/025370
(87) International publication number: WO 2007/011353

(56) References cited:
- EP-A- 0 448 891
- EP-A- 0 852 132
- WO-A-00/66031
- US-A- 6 152 947
- US-B1- 6 245 012
- US-B1- 6 443 972
- US-B2- 7 001 406

## Description

### 1. Field of the Invention

The present invention relates generally to a device for preventing the undesired passage of emboli from a venous blood pool to an arterial blood pool. The invention relates especially to a device for treating certain cardiac defects, especially patent foramen ovales and other septal defects through the use of an embolic filtering device capable of instantaneously deterring the passage of emboli from the moment of implantation.

### 2. Description of Related Art

The fetal circulation is vastly different than the normal adult circulation. The blood circulating in a fetus is oxygenated by the placenta, not the developing lungs. Therefore, the fetal circulation directs only a small percentage of the circulating blood to the fetal lungs. Most of the circulating blood is shunted away from the lungs to the peripheral tissues through specialized vessels and foramens that are open ("patent") during fetal life. In most people these specialized structures quickly close after birth. Unfortunately, they sometimes fail to close and create hemodynamic problems that can be fatal if left untreated.

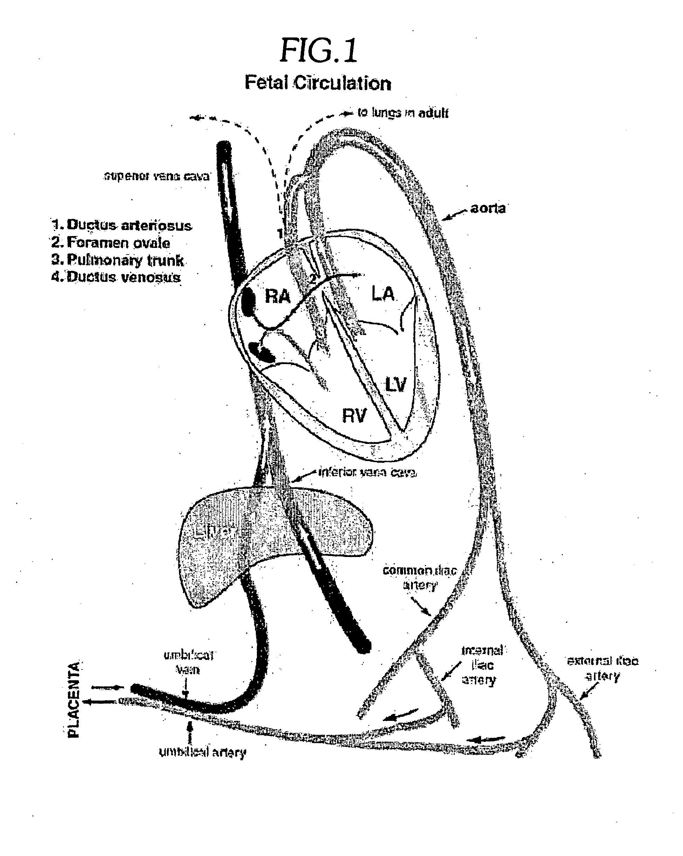

A diagram showing the blood circulation of a human fetus is illustrated in FIG. 1. The umbilical arteries branch off of the iliac arteries and deliver unoxygenated blood to the placenta. The fetal blood travels through the capillary bed in the placenta and transfers carbon dioxide to the maternal blood and takes oxygen and other nutrients from the maternal blood. The umbilical vein returns oxygenated blood to the fetus. Most of the oxygenated blood from the umbilical vein bypasses the developing liver and travels through a specialized vessel called the ductus venosus to the inferior vena cava and then into the right atrium. A good portion of the oxygenated blood from the inferior vena cava is directed across the right atrium and into the left atrium through a specialized curtain like opening in the heart called the foramen ovale. The blood from the left atrium then enters the left ventricle and then into the aorta where it travels to the head and other body tissues delivering the needed oxygen and nutrients.

The small amount of blood entering the right atrium that does not pass through the foramen ovale, most of which comes from the superior vena cava, flows into the right ventricle and then gets pumped into the pulmonary trunk and pulmonary arteries. Some of this blood is pumped into the developing lungs. However, the fetal lungs are collapsed which causes a high resistance to blood flow. Another specialized vessel, called the ductus arteriosus, is a vessel that connects the high pressure pulmonary artery to the lower pressure aorta. Therefore, most of the blood in the pulmonary artery flows into the lower pressure aorta through this specialized vessel.

Upon birth, the circulatory system goes through profound changes. The flow through the umbilical arteries and umbilical vein stops and consequently the flow through the musculature around the ductus venosus constricts and the blood flow through the ductus venosus stops. The lungs fill with air and the resistance to blood flow into the lungs drastically decreases. The corresponding pressure in the right atrium, right ventricle, and pulmonary arteries also decrease. The decrease in pressure in the right atrium causes the curtain like opening of the foramen ovale to close, driving more blood into the right ventricle and then to the lungs for oxygenation. Over time, the foramen ovale is replaced with a membrane called the fossa ovalis. Similarly, the decrease in pressure in the pulmonary arteries reduced the pulmonary arterial pressure to the same as or slightly less than the pressure in the aorta, which stops or reverses the flow through the ductus arteriosus. Once the muscular tissue of the ductus arteriosus is perfused with well oxygenated blood, the muscle begins to constrict and close the ductus arteriosus. The ductus arteriosus normally closes within about one week of life.

Usually over time, the unique openings of the fetal circulation become obliterated and a solid mass of tissue forms where these opening once were. However, in some people the opening remain. A patent ductus venosus after birth is very rare and almost always fatal. A patent ductus arteriosus occurs in about 1 out of every 5000 births. The patent ductus arteriosus once diagnosed is either medically treated or surgically ligated to close the ductus. In about one of four people, the foramen ovale does not seal shut, instead it remains patent. Such defects usually measure 10 mm or more in diameter and occupy one third or more of the length of the atrial septum in echocardiographic four chamber sections. Since the pressure in the left atrium is about two to four mm Hg greater than the pressure in the right atrium, the curtain like opening usually remains shut. However, if the pressure in the right atrium increases, such as upon heavy lifting or while performing a Valsalva type maneuver, the curtain like fold of tissue opens and the blood flows from the right atrium to the left atrium.

Studies have shown that adults with strokes of unknown origin, i.e., cryptogenic strokes, have about twice the normal rate of patent foramen ovales than the normal population. Although there is a correlation between strokes and patent foramen ovales, it is currently unknown why this correlation exists. It is theorized that blood clots and plaque that have formed in the peripheral venous circulation (in the legs for example) break off and travel to the heart. Normally, the clots and plaque get delivered to the lungs where it is tapped and usually cause no harm to the patient. Patients with a patent foramen ovale, however, have a potential opening that the clots or plaque can pass through the venous circulation and into the arterial circulation and then into the brain or other tissues to cause a thromboembolic event like a stroke. The clots may pass to the arterial side when there is an increase in the pressure in the right atrium. Then the clots travel through the left side of the heart, to the aorta, and then to the brain via the carotid arteries where they cause a stroke and the associated neurological deficits.

A number of atrial septal defects (ASD) closure devices have been developed and investigated in an attempt to develop a nonsurgical, transvenous method of occlusion of . ASD. These include the Sideris Buttoned device, the Angel Wing Das device, the atrial septum defect occlusion system (ASDOS) device, the Amplatz Septal Occluder, the CardioSEAL/StarFlex devices, and the Gore/Helix devices. Unfortunately, each of these devices have distinct disadvantages and limitations ranging from the size of the device delivery sheath, ease of implantation, feasibility, safety and effectiveness. The Sideris buttoned device is made of a polyurethane foam occluder with a Teflon coated wire skeleton, which is positioned within the left atrium, and a polyurethane foam rhomboid shaped counteroccluder with a Teflon coated wire skeleton, which is positioned in the right atrium. The major disadvantage with this device is the lack of a centering mechanism. For this reason, use of the devices at least two times the size of the stretched ASD is required. (Sievert H. Koppeler P. Rux S: Percutaneous closure of 176 interarterial defects in adults with different occlusion devices-6 years of experience [abstract], J. Am. Coll. Cardiol 1999, 33:519A.) Consequently, closure of defects may become difficult because the required size may be too large for the atrial septum to accommodate, or the device may impinge critical structures. There are also reports that the retrieval of the Sideris button device after incorrect deployment is difficult. (See, e.g., Rigby, Michael L., The Era of Transcatheter Closure of Atrial Septal Defects, Heart; 81:227-228 (1999)).

The "Angel Wings" device comprises two square frames made of superelastic Nitinol wire, each square frame having four legs that are interconnected by flexible islets at the corners. The wire frames are covered by polyester fibers. There is a conjoint suture ring of the right and atrial discs, which allow self centering on deployment. The device is delivered through an 11-13 F Mullins sheath. The major disadvantage of using this device is the attendant risk of aortic perforation cause by its sharp eyelet corners. In fact, the Angel Wings device was withdrawn from further clinical trials because of this problem. . (Syamaxundar Rao, P., M.D., Summary and Comparison of Atrial Septal Defect Closure Devices, Current Interventional Cardiology Reports 2000, 2:367-376 (2000)). The device is also ill-suited for treating fenestrated defects.

The atrial septal defect occlusion system (ASDOS) prosthesis (Microvena Corp., White Bear Lake, MN) consists of two umbrellas made of Nitinol and a patch of porous polyurethane attached to the left and right atrial devices. The device is introduced transvenously over a long veno-arterial guidewire and through an 11 F venous transeptal sheath. While the device is retrievable in the event of malpositioning before release of the device, it requires a complex procedure to implant, and the components are known to have a high incidences of thrantosis. It is also reported that frame fractures have been detected in 20% of the patients treated with this device.

The Amplatzer device is the subject of U.S. Patent No. 5,944,738 to Amplatzer, et al. This device is a saucer-shaped device formed from a mesh of fine Nitinol wires with a central connecting cylinder having a diameter similar to that of the stretched diameter of the defect. Thrombosis following implantation of the device is induced by three polyester patches. The device is delivered through a 6-10 F Mullins sheath. The primary disadvantage with this device is that it is ill-suited for closing fenestrated defects. Moreover, the device is a thick, bulky profile which dramatically increases the chances that the device will interfere with the heart's operation. Another disadvantage is its known capacity for incomplete endothelialisation with thrombus formation.

The CardioSEAL® device (NMT Medical, Inc.) is the subject of U.S. Patent No. 6,206,907 to Marino, et al. This occlusion device is comprised of a center section to which stranded wire elastic shape memory fixation devices are attached. The fixation devices hold the occlusion devices in place once it is inserted into an aperture. Attached to the fixation devices are polyvinyl foam sheets which occlude the aperture. While the CardioSEAL is deemed to be relative easy to use, it is reported that, of all the devices, the CardioSEAL device has the highest incidence of arm fractures, which has raised serious issues concerning its safety. Moreover, the CardioSEAL device, like the Amplatzer device is relatively large, and requiring at least a 10 F or 11 F delivery systems, and an undue amount of hardware within the heart. These characteristics increase the chance that the device will interfere with the heart's operation, lend to residual shunting and/or embolization. The size of the CardioSEAL device also renders it less suitable for small children.

The STARflex® device (NMT Medical, Inc.) is an updated version of the CardioSEAL device, which includes a self-centering mechanism consisting of four flexible springs which pass between the two fabric disks. While this added feature may reduce the instances of residual shunting, the aforementioned defects and disadvantages of the CardioSEAL are still a concern.

In view of these drawbacks and related-risks, the method of choice to close a patent foramen ovale is still open heart surgery and ligation of the foramen ovale to close it. Surgery, however, is obviously associated with the usually risks of general anesthesia, open heart procedures, infections, etc. Thus, there is a need for a safe, cost-effective, and easily implantable device for preventing the passage of emboli from an arterial blood pool and a venous blood pool which is not subject to the defects and disadvantages of known devices.

EP-A-0 852 132, upon which the preamble of claim 1 is based, discloses a blood filter for positioning within a blood vessel to trap blood clots. The filter includes a head to which there are attached and from which there extends several legs comprising at least one elongated element having two opposite ends. The legs are radially moveable and the elongated element of at least some of these legs has a shape folded back upon themselves, substantially in the form of a loop. In order to assist attachment of the device to the wall of the vessel, hooks can be set alternately in the direction towards the proximal end and the distal end, in order to ensure that the filter is held securely in both directions.

WO-A-00166031 discloses a blood filter that is convertible from a filter configuration to an open stent-like configuration. The filter includes a plurality of intraluminal filter elements (filter legs) that may be formed into a single cone or dual cone filter structure. A retainer secures the filter legs in the filter configuration upon initial deployment within a vessel. The retainer is then either self-releasing or removable to permit the filter legs to expand from the filter configuration into what may generally be described as an open or stent-like configuration. A filter web extends, at least in part, between the filter legs.

### SUMMARY OF THE INVENTION

According to the present invention there is provided the embolic filtering device of claim 1.

The embodiments of embolic filtering device described and illustrated hereinafter are relevant to treating septal defects, including patent foramen ovales. In one preferred embodiment particularly suited for treating patent foramen ovales, the embolic filtering device comprises an embolic filter, composed of metal, fiber, and/or polymer, for preventing the passage of emboli through the septal defect, and a frame which allows the device to be secured within and or adjacent to the lumen of the septal defect.

The embolic filter is made by, for example, (1) swaging one end of a piece of tubular mesh at a first end with a first fastener (2) pulling the free end of the mesh over the first fastened end so that it overlaps the first portion; (3) swaging a second, center section of the tubular section to form a 3-dimensional ball-like structure having a first diameter portion with a second fastener, (4) extending the remaining free end of the tubular mesh back over the 3 dimensional ball-like structure of the first and second portions of the tubular mesh; and (4) swaging the free end of the tubular mesh with a third fastener to form an exterior 3-dimensional ball-like structure having a second diameter portion, within which the 3-dimensional ball-like structure of first diameter portion is disposed.

The mesh is removably is secured to at least one or more bases of the frame, and positioned between the arms thereof. In a preferred embodiment, the bases of the frame and the fasteners which secure the tubular mesh are collars, having central lumens. The aforementioned third-fastener is insertable into the lumen of at least one of the bases of the frame in order to secure the mesh to the frame. The lumens of the fasteners and bases are aligned along a common axis in order that a the embolic filtering device can be loaded onto a guide wire.

In an exemplary embodiment, the frame, preferably composed of metal, fabric and/or a polymer, includes at least one base and at least two arms which extend therefrom, between which the mesh is at least partially disposed. The arms are positioned opposite one another and, in their resting state, are spaced apart from one another. When, as in a preferred embodiment, the device is composed of a shape memory metal, such as nitinol, the device can is be collapsed into a catheter tube by compressing the arms of the frame toward one another, causing the length of the device to increase, and the width to decrease. As the device is released from the catheter tube, it reverts to its functional, relaxed state. The embolic filtering device may also be composed of non-shape memory metals, such as elgiloy, cobalt chromium, and stainless steel, for example. Each arm includes at least one anchor positioned on the arms of the frames. The anchors can either be arcuate or linear in formation, depending on the shape of the patent foramen ovale to be treated, and are of sufficient rigidity to secure the device within the lumen of a septal defect.

To allow for non-invasive visualization of the device within a subject at least a portion of the frame or mesh is composed of or coated with a radiopaque material, such as tantalum. The device may also be treated with thrombin, collagen, hyluron, or a host growth factor to encourage and facilitate growth of tissue onto the device so as to further secure the device within the septal defect. The device can also be coated with an anticoagulant to deter formation of blood clots on the surface of the device.

In an exemplary embodiment, the mesh is composed of at least 96 strands of .002" diameter wire braided such that the wires are situated at an angle of 35° relative to the longitudinal axis of the device. The interstices created by the braided wires are small enough such as to effectively filter emboli, thereby preventing emboli from passing through the patent foramen ovale, or other septal defect.

### BRIEF DESCRIPTION OF THE DRAWINGS

FIG. 1 is a schematic diagram of the fetal circulation;

FIG. 2A illustrates a preferred embolic filtering device;

FIG. 2B illustrates another preferred embolic filtering device;

FIG. 2C illustrates a top view of the embolic filtering device illustrated in FIG. 2B;

FIG. 2D illustrates a preferred frame of the embolic filtering having two bases;

FIG. 3 illustrates another preferred embolic filtering device with a frame having one base;

FIG. 4 illustrates a preferred embolic filtering device and delivery mechanism;

FIG. 5A illustrates another preferred embolic filtering device;\

FIG. 5B and 5C illustrate a preferred embolic filtering device within a patent foramen ovale;

FIGS.6A and 6B illustrate another preferred embolic filter device; and

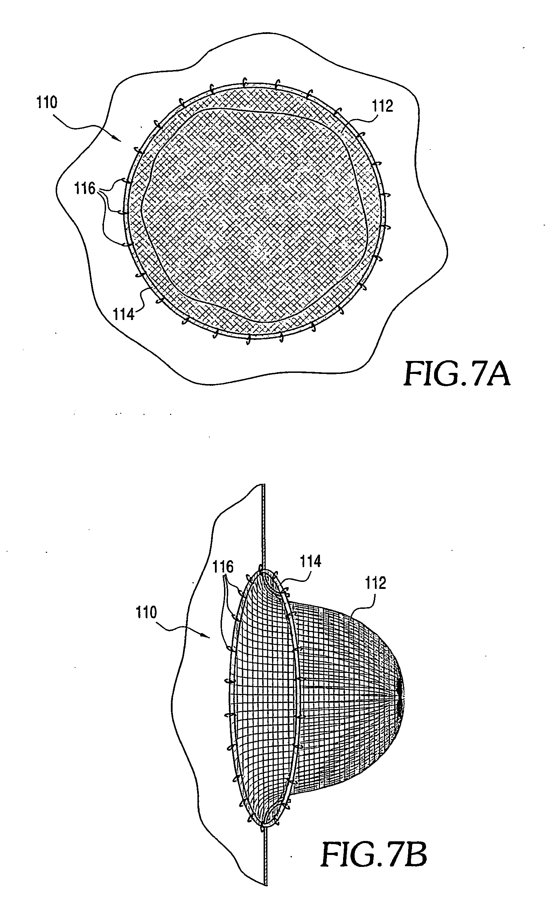

FIGS 7A and 7B illustrated another preferred embolic filter device.

### DETAILED DESCRIPTION OF THE INVENTION

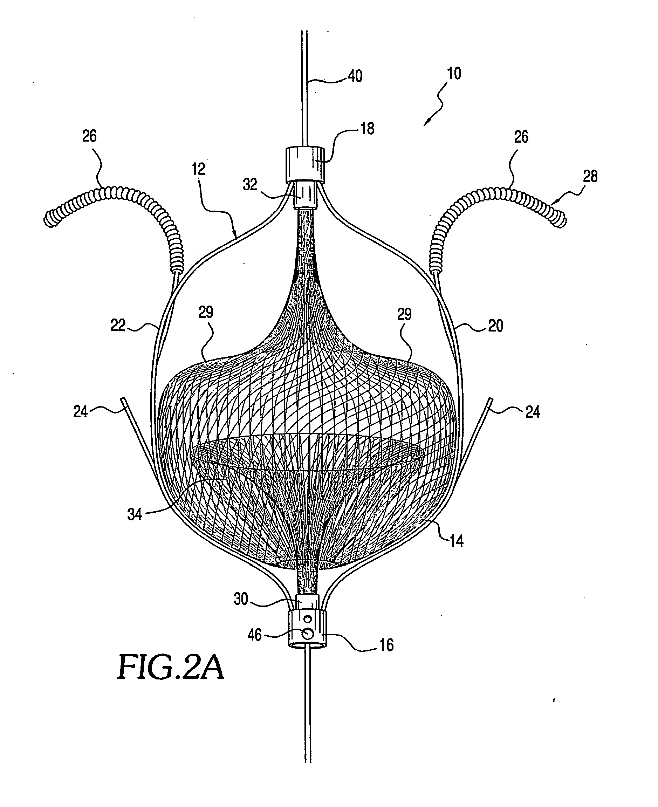

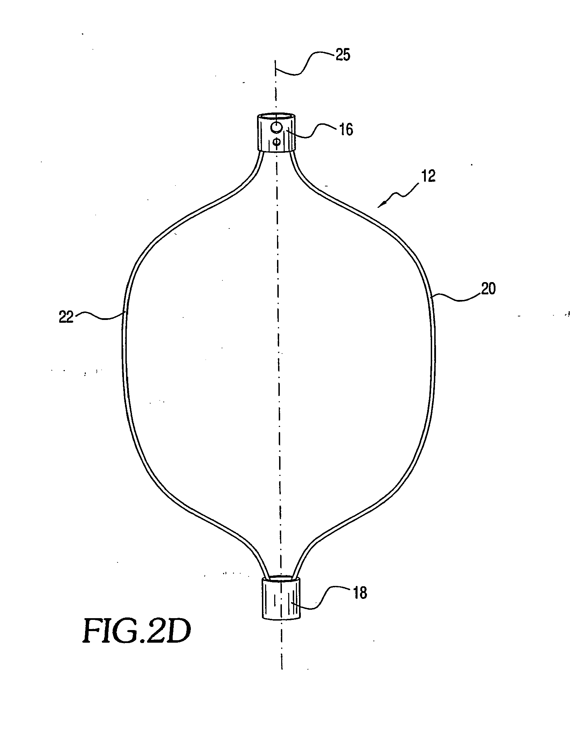

The present invention is directed generally to apparatus for preventing the passage of emboli between a venous blood pool and an arterial blood pools using devices for creating a barrier to the conducting of emboli at a passage between a venous blood pool and an arterial blood pool. The device is particularly suitable for treating cardiac defects, such as patent foramen ovale or other atrium septal defects. In a referred embodiment, exemplified at FIG. 2A, an embolic filtering device 10 is provided comprising a frame 12 and an embolic filter 14 comprising a mesh of stranded fabric, wire, or polymer. FIG. 2D illustrates one embodiment of frame 12 without embolic filter 14 attached. 3n this embodiment, frame 12 consists of a first base 16 and a second base 18. Each end of arms 20 and 22 are connected to first base 16 and second base 18, such that the lumens of first base 16 and second base 18 are in line with longitudinal axis 25 of frame 12. Arms 20 and 22 are preferably formed of a shape memory metal, e.g., nitinol, and formed such that, in the resting state, they are spaced apart from one another.

Referring to FIG. 2A, extending laterally from each of arms 20 and 22 proximate to first base 16 are right anchors 24. Right anchors 24 can be of any shape or formation suitable for delivering embolic filtering device 10 to the desired location and securing it in place. In a preferred embodiment, right anchors 24 are preferably linear or arcuate, and extend outward from frame 12 and away from first base 16, in the direction of second base 18, at an acute angle relative to longitudinal axis 25. The desired length of right anchors 24 and the position from which they extend from arms 20 and 22 will depend primarily on the size of the passage or defect to be treated. In any event, the right anchors 24 are of sufficient length to securely engage tissue within and/or adjacent to the septal defect. For example, when treating a patent foramen ovale, right anchors 24 preferably engage tissue within and/or adjacent to the right-atrial opening of the patent foramen ovale. Extending arcuately and/or laterally from the portion of arms 20 and 22 proximate second base 18 are left anchors 26. Left anchors 26 can be of any shape or formation suitable for delivering embolic filtering device 10 to the desired location and securing it in place; however, it has been found that arcuate or coiled anchors are most suitable for effectively securing the device within the area of interest. As with right anchors 24, left anchors 26 are of sufficient length to securely engage tissue within and/or adjacent to the septal defect to be treated. For example, when treating a patent foramen ovale, left anchors 26 preferably engage tissue within and/or adjacent to the left-atrial opening patent foramen ovale. In a preferred embodiment, right anchor 24 and left anchor 26 are covered with tantalum coil 28, or other radiopaque material, to allow for visualization of the position and location of embolic filtering device 10 after implantation in a subject. First base 16 and second base 18 and, for that matter, any portion of device 10 can likewise be compromised of radiopaque materials to provide even more visual points of reference in the imagery of embolic filtering device 10.

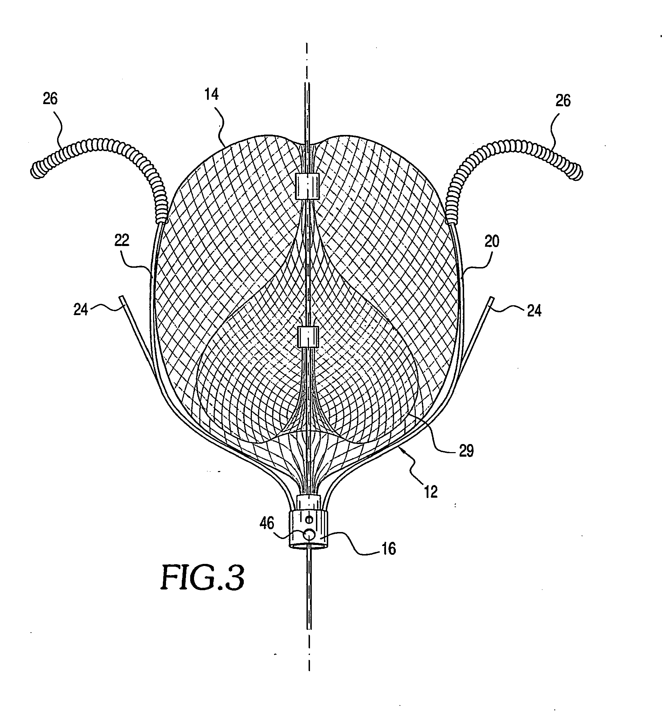

In another embodiment illustrated in FIG. 3, provided is a frame 12 having first base 16, but without second base 18, and shortened arms 20 and 22. By eliminating second base 18, the amount of hardware implanted in the passage to be treated is minimized. Since, as discussed below, second base 18 resides closest to the left atrium of the heart when embolic filtering device 10 is used to treat a patent foramen ovale, eliminating second base 18 minimizes the amount of hardware adjacent to or within the left atrium, decreasing the chance the operation of the left atrium will be comprised, and reducing the surface area upon which blood clots can form.

Embolic filter 14 is removably coupled to frame 12, and is preferably comprised of plurality of braided wire strands having a predetermined relative orientation and interstitial space between the strands. Those skilled in the art will appreciate that the number and diameter of the wires used may be varied to achieve the desired density and stiffness of the fabric, and the known size of the emboli sought to be filtered. In a preferred embodiment, the wire mesh consists of at least 96 strands of 0.002" diameter wire, situated at an angle of approximate 35° relative to the longitudinal axis 25. Suitable wire strand materials may be selected from a group consisting of a cobalt-based low thermal expansion alloy referred to in the field as "Elgiloy," nickel-based high temperature high-strength "superalloys" (including nitinol), nickel-based treatable alloys, a number of different grades of stainless steel, and polymers, including polyester, nylon, polytetrafluoroethylene (PTFE), polyurethane, polyaryletheretherketone (PEEK), and polyglycolic acid (PGA), polylactide (PLA), polyepsilon-caprolactone, polyethylacrylate (PEA). Platinum and alloys of platinum can also be co-braided, co-knitted or co-woven into mesh 14 to assist in determining where mesh is positioned within the patent foramen ovale. In a preferred embodiment, the wire strands are made from a shape memory alloy, NiTi (known as nitinol) which is an approximately stoichiometric alloy of nickel and titanium and may also include minor amounts of other metals to achieve desired properties. The frame 12 of device 10, and its components, including base 16, base 18, right arms 20 and left arms 22, are also preferably manufactured from so-called shape memory alloys. Such alloys tend to have a temperature induced phase change which will cause the material to have a preferred configuration which can be fixed by heating the material above a certain transition temperature to induce a phase change in the material. When the alloy is cooled, the alloy will "remember" the shape it was in during the heat treatment and will tend to assume that configuration, unless constrained from doing so.

Handling requirements and variations of NiTi alloy compositions are known in the art. For example, U.S. Patent Nos. 5,067,489 (Lind) and 4,991,602 (Amplatz et al.), discuss the use of shape memory NiTi alloys in guide wires. Such NiTi alloys are preferred, at least in part, because they are commercially available and more is known about handling such alloys than other known shape memory alloys. NiTi alloys are also very elastic and are said to be "superelastic" or "pseudoelastic." This elasticity allows device 10 to return to a preset configuration after deployment from a catheter or other delivery device. The relaxed configuration is generally defined by the shape of the fabric when it is deformed to generally conform to the molding surface of the mold in which it was created. The wire stands are manufactured by standard braiding processes and equipment.

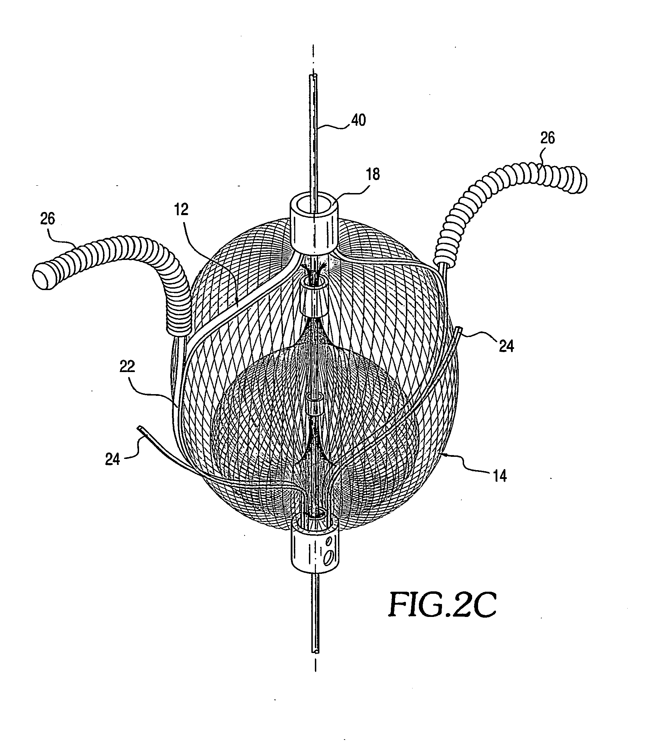

Embolic filter 14 of the present invention is preferably in the shape of a three-dimensional ball or sphere, as exemplified in FIGS. 2A and 2C. Starting with a tubular piece of braided mesh or the like, the three-dimensional ball or sphere, as exemplified in FIG. 2A, is, for example, made by swaging a first end of the mesh with a first fastener 30, and pushing said first fastener 30 upwards into the lumen of the tubular mesh, to create interior lobes 29. A center portion of the mesh is then swaged with a second fastener 32, creating an interior embolic filter portion 34. The remaining mesh is then extended back over said first fastener 30 and interior embolic filter portion 34, and the second end of the braided tubular mesh is swaged with a third fastener 36. First fastener 30, second fastener 32, and interior embolic filter portion 34 are in effect situated within exterior embolic filter portion 38. Third fastener 36 is situated outside of said exterior embolic portion 38. In a preferred embodiment, fasteners 30, 32 and 36 are collars having a central lumen. The lumens of the collars are substantially aligned along a common longitudinal axis 24, and dimensioned to receive a guide wire 40. Embolic filter 14 is preferably secured to frame 12 by inserting third fastener 36 into the lumen of first base 16 of frame 12. To reduce the chance of third fastener 36 from disengaging from first base 16, third fastener 36 and first base 16 can be coupled together, either by a mechanical locking means such as that created by a press fit, a melted polymer interlock, or hot melt adhesive, or by plasma welding. Plasma welding is the preferred coupling method, as it allows first base 16 to be shorter, since no portal is required on the base. When coupled to frame 12, embolic filter 14 resides at least partially between arms 20 and 22, such that the lumens of fasteners 30, 32, and 36 are substantially aligned with the lumens of first base 16 and second base 18 (if employing a frame with second base 18), along longitudinal axis 25. A plug composed of collagen, fabric, an adhesive, polymer or foam, for example, may be disposed within the aforementioned sphere to further deter the passage of embolic through the mesh.

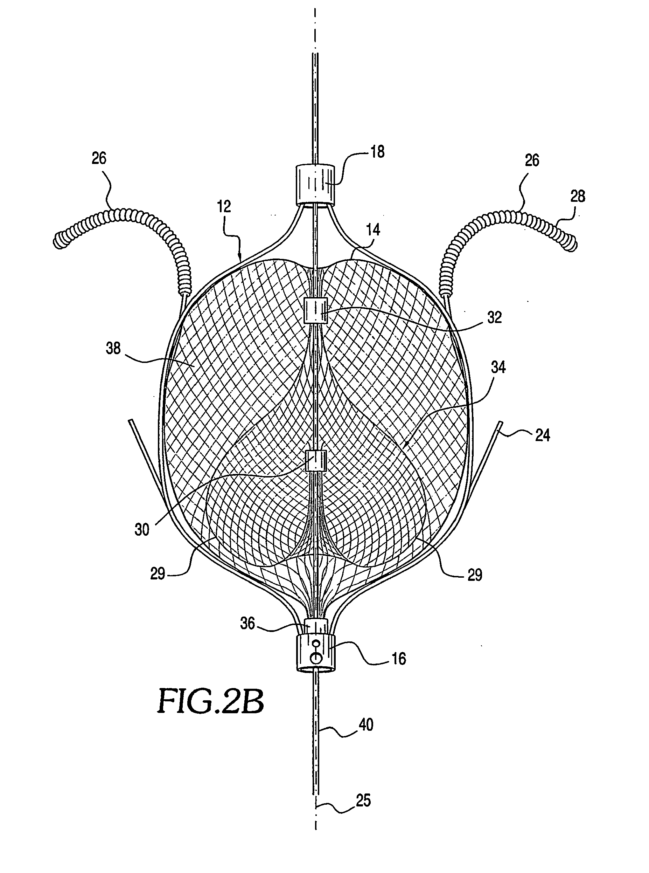

In another preferred embodiment, illustrated in FIG. 2A, an embolic filter 14 is provided which, instead of having a spherical shape as exemplified in FIGS. 2B and 3, has a first end comprising at least one lobe-like formation and a second end which tapers inward therefrom.. To make this embodiment, a piece of tubular mesh of suitable length, for example, is swaged at a first end by a first fastener 30. This first fastened end is then pushed into the lumen of the tubular mesh to form lobes 29. The second end of the mesh is then swaged by a second fastener 32. This embodiment is attached to frame 12 by securing first fastener in the lumen of base 16, and securing second fastener 32 in the lumen of base 18. As discussed above, fasteners 30 and 32 are collars having central lumens. The lumens of the collars are substantially aligned along a common longitudinal axis, and dimensioned to receive a guide wire 40.

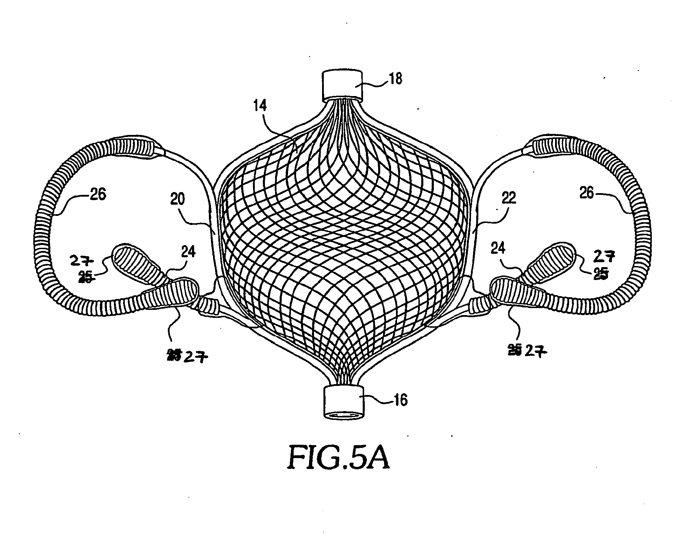

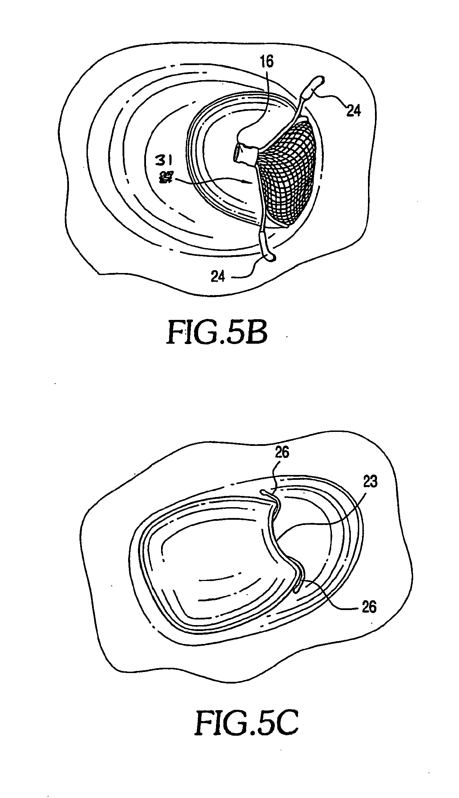

In another preferred embodiment, illustrated in FIG 5A, provided is an embolic filtering device 10, similar to those embodiments described above, but having right anchors 24 which are specifically designed to engage the perimeter of the tissue defining the right-atrial opening 23 of the patent foramen ovale, as illustrated in FIG. 5B. Contrary to right anchors 24 discussed in the aforementioned figures, the ends of right anchors 24 of this embodiment reside against or adjacent to the outside of the tissue wall defining the patent foramen ovale. Right anchors 24 are, therefore, preferably of slightly longer dimension and at least slightly arcuate in shape to facilitate this methodology. The ends of right anchors 24 in this embodiment, include protective caps 27 at their distal ends. Caps 27 can be composed of rubber, plastic, or any other suitable material for covering the ends of anchors 24 and 26, and may also comprise radiopaque materials, for example, in order to allow post-implant visualization of the location and positioning of anchors 24 after implant.

It will be recognized by those of ordinary skill that the manner in mesh 14 can be manufactured in a variety of ways without departing from the scope of the invention. For example, it will be recognized that mesh 14 does not necessarily need to be spherical, or have both an interior and exterior embolic portion, as discussed above. Mesh 14 can be of any shape and dimension suitable to deter the passage of embolic material between a venous blood pool and an arterial blood pool, and can include any number of layers, so long as the interstices between the strands forming mesh 14 are of sufficient area to filter emboli.

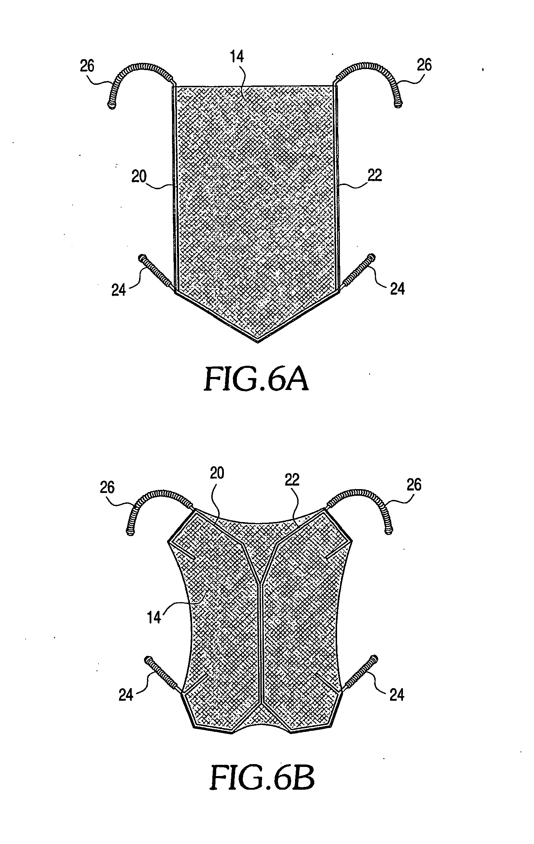

The design and dimensions of frame 12 can also be manufactured in a variety of ways without departing from the scope of the invention. FIG. 6A and 6b illustrate yet a further embodiment of the invention, wherein arms 20 and 22 are effectively decoupled from one another, such that the tissue distension function of embolic filtering device 10 is provided separately by each individual legs of the device. This allows embolic filtering device 10 to be more compact, and to better fill gaps and meet the contours of the patent foramen ovale. Particularly with respect to the embodiments shown in FIG. 6A and 6B, should be recognized that the size of mesh 14 need not be large, but can cover only arms 20 and 22 and still be effective in treating patent foramen ovales.

Device 10 provides distinct advantages and improvements over known patent-foramen ovale-treatment devices. First, the elasticity and ball-like structure of mesh 14, enables device 10 to treat a patent foramen ovales, or other septal defects, of any shape and dimension with equal effectiveness. This is because mesh 14 is compressible along its entire length. Thus, it does not matter if the patent foramen ovale is fenestrated, as the elasticity of mesh 14 will allow it to conform to the substantially exact shape and dimension of the patent foramen ovale. Mesh 14 can also be annealed to have a 3-dimensional to help fill any gaps within the patent foramen ovale space. Thus, the post-implant leakage along the perimeter of known devices caused by their inability to accommodate irregular shaped defects is eliminated. Second, device 10 has substantially less surface compared to known devices, thereby reducing the risk of dangerous blood clot formation on the exterior of the device. Third, contrary to known devices which do not prevent passage of emboli through the defect until tissue growth onto the device occludes the defect, the interstices between the stands of braided mesh 14 of the present invention are small enough to effectively filter emboli as soon as device 10 is implanted. Thus, device 10 offers immediate protection against the passage of emboli at the moment of implant.

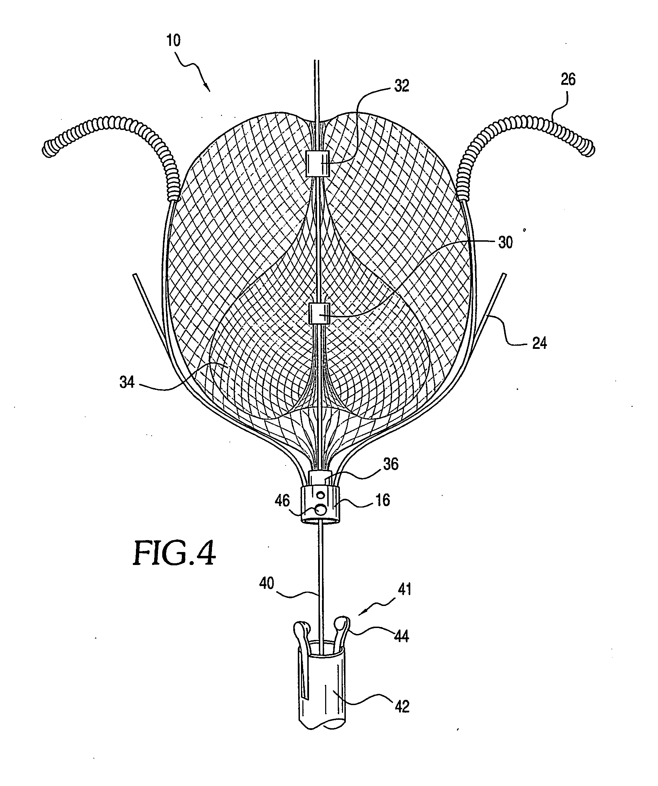

The embolic filtering device 10 is particular useful in preventing the passage of emboli between an venous blood pool and an arterial blood pool. For purposes of exemplary illustration, the method of the invention is herein exemplified through discussion of a method of treating a patent foramen ovale (PFO). However, it should be recognized that the invention can be used to prevent the passage of emboli between any septal defect and/or arterial venous blood pool and arterial blood pool. To deliver the embolic filtering device 10 of the patent foramen ovale, embolic filtering device 10 is loaded into a delivery system 41 comprising a catheter 42, exemplified in FIG. 4. In a preferred embodiment, the embolic filtering device 10 is loaded onto a guide wire 40 by inserting the guide wire through the lumens of first base 16, the lumens of fasteners 30, 32, and 36, if employing a frame 12 with second base 18, the lumen of second base 18. A pair of forceps 44, as exemplified in FIG. 4, or other grasping device, is used to grasp embolic filtering device 10. In a preferred embodiment, first base 16 has a recess 46 for receiving forceps 44, such that forceps 44 are positioned within recess 46 to more securely grasp embolic filtering device 10, and to deter embolic filtering device 10 from detaching from forceps 44. With embolic filtering device 10 secured by forceps 44 embolic filtering device 10 is pulled into catheter 42. As embolic filtering device 10 is pulled into catheter 42, the force of the catheter walls against first base 16 of frame 12 will force side walls 20 and 22, and left anchors 26 and right anchors 24 inward toward one another. Embolic filtering device 10 will gradually collapse as it is pulled into catheter 42.

Using catheter 42, embolic filtering device 10 is delivered to the patent foramen ovale, or other passage between a venous blood pool or arterial blood pool, to be treated. In particular, the distal end of catheter 42 is extended through the patent foramen ovale from the right atrial side to the left atrial side. With the distal end of catheter 42 positioned in the left atrium adjacent to the patent foramen ovale, forceps 44 are used to withdraw embolic filtering device 10 from catheter 42. As embolic filtering device 10 is withdrawn, embolic filtering device 10 will gradually expand from its collapsed position and into its memorized shape and/or in conformance to the shape and dimension of the patent foramen ovale being treated. With the distal end of catheter 42 positioned in the left atrium, adjacent to the patent foramen ovale, embolic filtering device 10 is withdrawn from catheter 42, while catheter 42 is slowly pulled back through the patent foramen ovale in the direction of the right atrium. Left anchors 26 are withdrawn first, and as catheter 42 is pulled back, left anchors 26 are caused to securely engage the walls defining the patent foramen ovale, preferably, the tissue defining the perimeter of the left-atrial opening 23 of the patent foramen ovale, as shown in FIG. 5C. As catheter 42 is pulled back further, the engagement of left anchors 26 onto the tissue defining the perimeter of the left-atrial opening 23 of arms 20 and 22 will prevent embolic filter device 10 from being pulled through the patent foramen ovale, and embolic filter 14 will emerge preferably within the patent foramen ovale, and will gradually expand apart from one another in returning to the shape memorized orientation. As arms 20 and 22 expand apart from one another, pressure will be exerted onto the tissue defining the lumen of the patent foramen ovale, thereby acting as a tissue distension device. The tissue defining the patent foramen ovale will naturally press inward against mesh 14, in effect squeezing the device within the patent foramen ovale. As catheter 42 is pulled back yet further, right anchors 24 will emerge and, as they expand to their memorized shape, will also forcibly engage, for example, the walls defining the patent foramen ovale, or the perimeter of the tissue defining right atrial opening 31 of the patent foramen ovale. If using the embolic filter device illustrated in FIG. 5A, for example, right anchors 24 will engage the tissue defining the outside perimeter defining the right-atrial opening 31 of the patent-foramen ovale, as illustrated in FIG. 5B. In its memorized shape, embolic filter 14 should be sized to engage the walls defining the patent foramen ovale with sufficient force to prevent emboli from passing between the exterior of the embolic filter 14 and the walls of defining the patent foramen ovale. Further, the force created from blood flowing from the right atrium to the left atrium against right anchors 24 facilitates the securing of right anchors 24, and helps prevent embolic filtering device 10 from becoming dislodged from its intended position.

It will be recognized by those of ordinary skill, that the device can further be secured in place by adhesives, sutures, hooks, barbs, or other such means. To enhance recovery subsequent to implanting embolic filtering device 10 frame 12 and/or mesh 14 can be coated with known drugs suitable for that purpose. Non-pharmacological methods can also be used to promote healing, including ultrasound, radiofrequency, radiation, mechanical vibration, or any other known non-pharmacological healing method.

Prior to disengaging embolic filtering device 10 from forceps 44 and removing catheter 42 from the subject, known radiological techniques can be employed to insure that embolic filtering device 10 is properly positioned and secured within the patent foramen ovale. If the position of embolic filtering device 10 needs to be altered, forceps 44, while still secured to embolic filtering device 10, can be used to reposition embolic filtering device 10; otherwise, forceps 44 are disengaged from embolic filtering device 10, and forceps 44, catheter 42, and guide wire 40 are withdrawn. Should embolic filter device 10 later become disengaged, disoriented, damaged or otherwise need to be removed, forceps 44 can be used to easily reposition or recover embolic filter device 10, as necessary. To facilitate the ease by which embolic filter device 10 is repositioned or recovered, base 16 is preferably coated with a suitable material to deter tissue from covering recess 46.

From the moment that embolic filtering device 10 is inserted, emboli are effectively filtered by embolic filtering device 10. Since blood travels from the direction of the right atrium to the left atrium, the portion of embolic filter 14 having a higher density of mesh, e.g., lobes 29 and/or interior embolic filter portion 34, are positioned on the right atria side to decrease the chances that emboli will penetrate into the left atrium. The design of embolic filtering device 10, however, is such that if emboli pass through the right side of embolic filter 14, there is still a significant chance that the portion of embolic filter 14 positioned on the left atrial side will prevent the emboli from passing into the left atrium.

Thus, unlike known devices for treating patent foramen ovale or atrial septal defects, for example, it is not necessary for thrombi to collect on the embolic filtering device 10 before the passage of emboli are effectively deterred. However, if total occlusion of the passage is desired, embolic filtering device 10 the embolic filter 14 can be treated with materials to promote thrombrosis, tissue in-growth, or adhesions. Embolic filter 14 can also be treated with anticoagulants to discourage blood clot formation on the device 10.

The primary function of frame 12 is to facilitate the delivery, positioning and securing of the embolic filter 14 within and/or adjacent to a passage between a venous blood pool and an arterial blood pool. It should be appreciated, however, that embolic filter 14 can be of virtually any shape that extend, out of the plane of the frame, such as spherical, round or oval, so long as it retains its ability to filter emboli.

The invention has been described through a preferred embodiment. However, those of ordinary skill will recognize that various modifications can be made without departing from the scope of the invention as defined by the claims.

## Claims

1. An embolic filtering device (10), positionable within a body opening, comprising:
a frame (12) comprising a first base (16), a first arm (20) and a second arm (22), wherein the first and second arms are coupled to the first base, and wherein the first arm comprises a first anchor (24), and wherein the second arm comprises a second anchor (24), and wherein the first arm is positioned substantially opposite the second arm, and wherein the first arm and the second arm are resiliently flexible, wherein the first arm further comprises a third anchor (26), and wherein the second arm further comprises a fourth anchor (26);
**characterized in that**:
the device further comprises a mesh (14) coupled to the frame;
the first anchor extends laterally away from the first base, the second anchor extends laterally away from the first base, the first anchor and third anchor are configured to anchor the device to tissue between the first and third anchors, the second anchor and the fourth anchor are configured to anchor the device to tissue between the second and fourth anchors; and
the frame is substantially planar and the mesh extends out of the plane of the frame.

2. The device of claim 1, further comprising a second base (18), wherein the first arm (20) connects the first base (16) to the second base.

3. The device of claim 1 or claim 2, wherein the first arm (20) is biased away from the second arm (22).

4. The device of any one of the preceding claims, wherein the mesh (14) comprises a metal.

5. The device of any one of the preceding claims, wherein the first anchor (24) and the second anchor (24) are arcuate.

6. The device of any one of claims 1 to 4, wherein the first anchor (24) and the second anchor (24) are linear.

7. The device of any one of the preceding claims, wherein each of the first anchor (24), the second anchor (24), the third anchor (26) and the fourth anchor (26) extends outward from the first and second arms (20, 22).

8. The device of any one of the preceding claims, wherein each of the first anchor (24), the second anchor (24), the third anchor (26) and the fourth anchor (26) has a free end.

9. The device of any one of the preceding claims, wherein the device (10) is collapsible into a catheter (42) and capable of expanding to a relaxed state as the device is released from the catheter, and wherein the length of the frame (12) is elongated when the first arm (20) and the second arm (22) are compressed perpendicularly to the longitudinal axis (25) of the frame.

## Patentansprüche

1. Emboliefiltervorrichtung (10), welche in einer Körperöffnung angeordnet werden kann, umfassend:
einen Rahmen (12), welcher eine erste Basis (16), einen ersten Arm (20) und einen zweiten Arm (22) umfasst, wobei der erste und der zweite Arm mit der ersten Basis gekoppelt sind, und wobei der erste Arm eine erste Verankerung (24) umfasst, und wobei der zweite Arm eine zweite Verankerung (24) umfasst, und wobei der erste Arm im Wesentlichen gegenüber dem zweiten Arm angeordnet ist, und wobei der erste Arm und der zweite Arm nachgiebig flexibel sind, und wobei der erste Arm darüber hinaus eine dritte Verankerung (26) umfasst, und wobei der zweite Arm darüber hinaus eine vierte Verankerung (26) umfasst;
**dadurch gekennzeichnet,**
**dass** die Vorrichtung darüber hinaus ein Sieb (14) umfasst, welches mit dem Rahmen gekoppelt ist;
**dass** sich die erste Verankerung seitlich weg von der ersten Basis erstreckt,
wobei sich zweite Verankerung seitlich weg von der ersten Basis erstreckt, wobei die erste Verankerung und die dritte Verankerung ausgestaltet sind, um die Vorrichtung an dem Gewebe zwischen der ersten und der dritten Verankerung zu halten, wobei die zweite Verankerung und die vierte Verankerung ausgestaltet sind, um die Vorrichtung an dem Gewebe zwischen der zweiten und der vierten Verankerung zu halten; und
**dass** der Rahmen im Wesentlichen eben ist und sich das Sieb aus der Ebene des Rahmens heraus erstreckt.

2. Vorrichtung nach Anspruch 1, darüber hinaus eine zweite Basis (18) umfassend, wobei der erste Arm (20) die erste Basis (16) mit der zweiten Basis verbindet.

3. Vorrichtung nach Anspruch 1 oder Anspruch 2, **dadurch gekennzeichnet, dass** der erste Arm (20) weg von dem zweiten Arm (22) geneigt ist.

4. Vorrichtung nach einem der vorhergehenden Ansprüche, **dadurch gekennzeichnet, dass** das Sieb (14) ein Metall umfasst.

5. Vorrichtung nach einem der vorhergehenden Ansprüche, **dadurch gekennzeichnet, dass** die erste Verankerung (24) und die zweite Verankerung (24) gebogen sind.

6. Vorrichtung nach einem der Ansprüche 1 bis 4, **dadurch gekennzeichnet, dass** die erste Verankerung (24) und die zweite Verankerung (24) geradlinig sind.

7. Vorrichtung nach einem der vorhergehenden Ansprüche, **dadurch gekennzeichnet, dass** sich die erste Verankerung (24), die zweite Verankerung (24), die dritte Verankerung (26) und die vierte Verankerung (26) jeweils von dem ersten und zweiten Arm (20, 22) nach außen erstrecken.

8. Vorrichtung nach einem der vorhergehenden Ansprüche, **dadurch gekennzeichnet, dass** die erste Verankerung (24), die zweite Verankerung (24), die dritte Verankerung (26) und die vierte Verankerung (26) jeweils ein freies Ende aufweisen.

9. Vorrichtung nach einem der vorhergehenden Ansprüche, **dadurch gekennzeichnet, dass** die Vorrichtung (10) in einen Katheter (42) zusammenlegbar ist und geeignet ist, um in einen entspannten Zustand ausgedehnt zu werden, wenn die Vorrichtung von dem Katheter gelöst wird, und dass sich die Länge des Rahmens (12) verlängert, wenn der erste Arm (20) und der zweite Arm (22) senkrecht zu der Längsachse (25) des Rahmens zusammengedrückt werden.

## Revendications

1. Dispositif de filtrage embolique (10), positionnable dans une ouverture corporelle, comprenant :
un cadre (12) comprenant une première base (16), un premier bras (20) et un deuxième bras (22), dans lequel les premier et deuxième bras sont couplés à la première base, et dans lequel le premier bras comprend un premier élément d'ancrage (24), et dans lequel le deuxième bras comprend un deuxième élément d'ancrage (24), et dans lequel le premier bras est positionné substantiellement en face du deuxième bras, et dans lequel le premier bras et le deuxième bras sont flexibles de façon résiliente, dans lequel le premier bras comprend en outre un troisième élément d'ancrage (26), et dans lequel le deuxième bras comprend en outre un quatrième élément d'ancrage (26) ;
**caractérisé en ce que** :
le dispositif comprend en outre un filet (14) couplé au cadre ;
le premier élément d'ancrage s'étend latéralement à l'écart de la première base, le deuxième élément d'ancrage s'étend latéralement à l'écart de la première base, le premier élément d'ancrage et le troisième élément d'ancrage sont configurés pour ancrer le dispositif dans un tissu situé entre les premier et troisième éléments d'ancrage, le deuxième élément d'ancrage et le quatrième élément d'ancrage sont configurés pour ancrer le dispositif dans un tissu situé entre les deuxième et quatrième éléments d'ancrage ; et
le cadre est substantiellement plan et le filet s'étend hors du plan du cadre.

2. Dispositif selon la revendication 1, comprenant en outre une deuxième base (18), dans lequel le premier bras (20) relie la première base (16) à la deuxième base.

3. Dispositif selon la revendication 1 ou 2, dans lequel le premier bras (20) est écarté du deuxième bras (22).

4. Dispositif selon l'une quelconque des revendications précédentes, dans lequel le filet (14) comprend un métal.

5. Dispositif selon l'une quelconque des revendications précédentes, dans lequel le premier élément d'ancrage (24) et le deuxième élément d'ancrage (24) sont arqués.

6. Dispositif selon l'une quelconque des revendications 1 à 4, dans lequel le premier élément d'ancrage (24) et le deuxième élément d'ancrage (24) sont linéaires.

7. Dispositif selon l'une quelconque des revendications précédentes, dans lequel chacun des premier élément d'ancrage (24), deuxième élément d'ancrage (24), troisième élément d'ancrage (26) et quatrième élément d'ancrage (26) s'étend vers l'extérieur depuis les premier et deuxième bras (20, 22).

8. Dispositif selon l'une quelconque des revendications précédentes, dans lequel chacun des premier élément d'ancrage (24), deuxième élément d'ancrage (24), troisième élément d'ancrage (26) et quatrième élément d'ancrage (26) a une extrémité libre.

9. Dispositif selon l'une quelconque des revendications précédentes, dans lequel le dispositif (10) peut être replié dans un cathéter (42) et est apte à se dilater jusqu'à un état relâché quand on libère le dispositif du cathéter, et dans lequel la longueur du cadre (12) est allongée quand le premier bras (20) et le deuxième bras (22) sont comprimés perpendiculairement à l'axe longitudinal (25) du cadre.
